# EUROPEAN PATENT APPLICATION

(11) **EP 4 582 490 A1**
(43) Date of publication of application: **09.07.2025**
(21) Application number: 23859875.9
(22) Date of filing: 19.07.2023
(51) Int. Cl.: C08L 101/00, C08F 8/06, C08F 20/34, C08F 20/36, C08F 26/06, C08L 39/00, C12N 1/00

(54) **BIOCOMPATIBLE POLYMER COMPOSITION AND USE THEREOF**

(30) Priority: 29.08.2022 JP 2022135524
(71) Applicant: Artience Co., Ltd., Chuo-ku Tokyo 104-8377 (JP)
(72) Inventor: OGIWARA Naoto, Tokyo 104-8377 (JP); KANAI Yuki, Tokyo 104-8377 (JP); TAO Fumiya, Tokyo 104-8377 (JP); BABA Koki, Tokyo 104-8377 (JP); TAKAHASHI Koyo, Tokyo 104-8377 (JP)
(74) Representative: SSM Sandmair
(86) International application number: PCT/JP2023/026392
(87) International publication number: WO 2024/048105

(57) **Abstract**

A biocompatible polymer composition having excellent handling properties that serves as a protein stabilizer having excellent protein stabilizing performance, a blocking agent having an excellent sensitizing effect, and a cell culture medium additive capable of culturing antibody-producing cells at excellent antibody productivity and cell activity is provided. Provided is a biocompatible polymer composition that contains a vinyl polymer (A) having an amine oxide group and a mass-average molecular weight of 5,000 or more and water, the surface tension of a 1% aqueous solution of which at 25°C is 30-50 dyne/cm.

## Description

### Technical Field

The present disclosure relates to a biocompatible polymer composition and the use thereof.

### Related Art

The higher-order structure of proteins is maintained through non-covalent weak polar interaction such as van der Waals interaction, hydrogen bond, and electrostatic interaction within the polypeptide chain, and various functions are exhibited by such higher-order structure. Such bonding changes due to various external factors (for example, temperature, pH, salt concentration, concentration of the protein itself, and the coexistence with surfactants, denaturants, divalent metals, proteases, etc. ), and the structure of the protein may be destroyed, resulting in a state where the original function is lost. Therefore, the stability of proteins can be described as the ability to maintain a functional higher-order structure.
For such reason, it is necessary to stabilize proteins in aqueous solutions, and the optimization of storage conditions, such as constant storage in a buffer solution at a predetermined temperature, as well as the use of stabilizers (e.g., other proteins such as albumin; polyhydric alcohols such as glycerol, polyethylene glycol, sucrose; amino acids such as glutamic acid, lysine; reducing agents such as glutathione, dithiothreitol; metal chelating agents such as EDTA; organic acid salts such as citric acid; heavy metal salts, substrates, coenzymes) alone or in combination are considered (Non-Patent Document 1). Particularly as stabilizers for synthetic compounds, for example, Patent Document 1 discloses a method using a polymer having a phosphorylcholine group, Patent Document 2 discloses a method using a polymer compound having a polyethylene glycol portion, Patent Document 3 discloses a method using a low molecular weight amine oxide compound, and Patent Document 4 discloses a method using a low molecular weight sulfobetaine compound. Additionally, Patent Document 5 discloses a method using a polymer amine oxide compound.
However, such methods may not be able to provide a satisfactory retention rate or a stable period for certain types of proteins, and further improvements are sought.

In bioassays, techniques that utilize specific adsorption by antigen-antibody reaction to detect and visualize DNA and proteins are widely known in methods such as Southern blotting, Western blotting, ELISA, and immunostaining. Antibodies also cause non-specific adsorption reactions with substances other than the target protein. To prevent such non-specific adsorption, pretreatment is performed to cover the detection and measurement target surface with a blocking agent, so as to prevent non-specific adsorption but not interfere with specific adsorption.
As the blocking agent, for example, Patent Document 6 and Non-patent Document 2 disclose biologically derived proteins such as normal serum, bovine serum albumin, gelatin, and skim milk. Additionally, Patent Documents No. 7, 8, and 9 disclose a surfactant such as TWEEN (registered trademark) 20, hydroxyalkyl cellulose, polyvinyl alcohol, and a copolymer of (meth)acryloyl morpholine with other monomers. In addition, Patent Document 10 and Non-Patent Document 3 disclose copolymers having a phosphorylcholine group in the side chain, and Patent Document 5 discloses a method using a polymer amine oxide compound. However, biologically derived proteins such as those disclosed in Patent Document 6 and Non-patent Document 2 possess a potential issue of being prone to performance variation. Moreover, compounds disclosed in Patent Documents 5, 7, 8, 9, and 10 are not satisfactory in terms of the sensitizing effect on the antigen-antibody reaction, and further improvements are sought. Additionally, the copolymer disclosed in Patent Document 10 exhibits productivity issues, such as requiring multiple reactions and associated refinements at the time of synthesizing raw material monomers.

In cell culture of animal cells, industrial-scale production of useful bioactive substances (hormones, neurotransmitters, cytokines, vitamins and minerals, enzymes, nucleic acids, etc.), including monoclonal antibodies, has become increasingly common through large-scale culturing. In such cases, animal sera such as fetal bovine serum (FBS) are frequently used as growth factors. Specifically, culture solutions in which synthetic basic media such as RPM1 1640, Eagle's MEM, and Ham's F12 are added with approximately 10-20% animal serum such as fetal bovine serum (FBS) are used in general. However, the supply of animal sera such as fetal bovine serum (FBS) is limited, and the cost is generally high. Accordingly, the cost for the media increases, resulting in a higher manufacture cost for bioactive substances. It also leads to the drawback of making it difficult to isolate the desired bioactive substance from the culture solution containing serum-derived proteins. Furthermore, in the sera, variations in quality can be observed between lots. Therefore, it is necessary to thoroughly perform lot check before using sera and select a usable serum, and such serum selection requires considerable efforts. In addition, regarding animal-derived sera, there are issues related to prion-related diseases such as mad cow disease, bovine spongiform encephalopathy, infectious spongiform encephalopathy, and Creutzfeldt-Jakob disease. Moreover, since animal-derived sera cannot be sterilized by autoclaving, there is a possibility of viral or mycoplasma contamination, leading to issues of risks such as infection (or risk of safety). To address these issues, studies on serum-free media have been conducted. For example, Patent Document 11 discloses that adding glycylglycine to the medium enables animal cells to produce useful substances at a high concentration. In addition, Patent Document 12 discloses that bioactive substances can be produced efficiently in the case where cells are cultured in a medium containing polymers having a phospholipid-like structure. Moreover, Patent Document 13 discloses that a medium containing a hydrophobic D-amino acid promotes cell proliferation and increases the production amount of useful substances. However, most of such media require further improvements in terms of proliferation, viability, and antibody productivity of antibody-producing cells. Furthermore, many serum substitutes are derived from natural sources whose supply is limited and cost generally high. Therefore, the development of serum substitutes to replace the fetal bovine serum (FBS) is anticipated. Patent Document 5 discloses that cell aggregate formation is favorable and the proliferation and viability of antibody-producing cells increase in the case where cells are cultured in a medium having a polymer amine oxide compound. However, there is room for improvement in the performance of the antibody-producing cells, and there is an issue that the handling properties due to foaming deteriorate.

### Citation List

### Patent Literature

Patent Document 1: Japanese Unexamined Patent Application Publication No. H10-045767
Patent Document 2: Japanese Unexamined Patent Application Publication No. H10-237094
Patent Document 3: Japanese Translation of PCT International Application Publication No. JP-T-2007-509164
Patent Document 4: Japanese Translation of PCT International Application Publication No. JP-T-2007-516281
Patent Document 5: Japanese Unexamined Patent Application Publication No. 2020-38059
Patent Document 6: WO2016/052690
Patent Document 7: Japanese Unexamined Patent Application Publication No. 2004-219111
Patent Document 8: Japanese Unexamined Patent Application Publication No. H04-019561
Patent Document 9: Japanese Unexamined Patent Application Publication No. 2008-209114
Patent Document 10: Japanese Unexamined Patent Application Publication No. H07-083923
Patent Document 11: Japanese Unexamined Patent Application Publication No. H07-023780
Patent Document 12: Japanese Unexamined Patent Application Publication No. 04-304882
Patent Document 13: Japanese Unexamined Patent Application Publication No. 2015-027265

### Non-patent Literature

Non-patent Document 1: Course on New Biochemistry Experimentation I, Protein I, Separation, Refinement, and Properties, Chapter 16
Non-patent Document 2: "Watanabe-Nakane Enzyme Immunoassay" published by Interdisciplinary Planning Co., Ltd., 2002
Non-patent Literature 3: KOBUNSHI RONBUNSHU, Vol. 35, No. 7, p. 423 (1978)

### SUMMARY OF INVENTION

### Technical Problem

In view of the above, an objective of the disclosure is to provide a biocompatible polymer composition having excellent handling properties that serves as a protein stabilizer having excellent protein stabilizing performance, a blocking agent having an excellent sensitizing effect, and a cell culture medium additive capable of culturing antibody-producing cells at excellent antibody productivity and cell activity.

### Solution to Problem

That is, the disclosure relates to [1] to [14] below.
[1] A biocompatible polymer composition, comprising: a vinyl polymer (A) and water, the vinyl polymer having an amine oxide group and a mass-average molecular weight of 5,000 or more, wherein a surface tension of a 1% aqueous solution at 25°C is 30 dyne/cm to 50 dyne/cm.
[2] In the biocompatible polymer composition, an osmotic pressure in the 1% aqueous solution is 1 mOsm/kg to 20 mOsm/kg.
[3] In addition, the biocompatible polymer composition further includes a compound (B), excluding a surfactant and a monomer, having an amphoteric ion structure, and exhibiting a molecular weight of 500 or less.
[4] In the biocompatible polymer composition, a proportion of the compound (B) is 0.5 parts to 10 parts with respect to 100 parts of the vinyl polymer (A).
[5] In the biocompatible polymer composition, the vinyl polymer (A) having an amine oxide group and a mass-average molecular weight of 5,000 or more has at least one structure represented by General formulas 1 to 3 below: 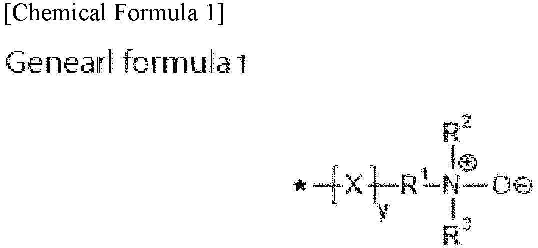 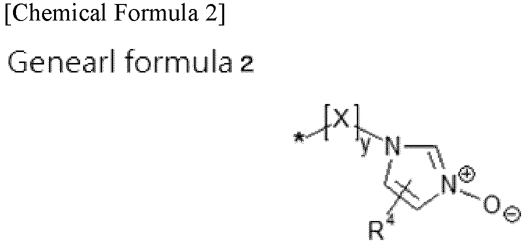 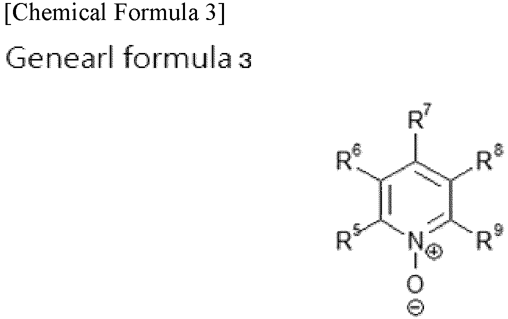 [In General formulas 1 to 3,
   X represents a divalent linking group,
   y represents 0 or 1,
   R¹ represents an alkylene group having 1 to 6 carbon atoms,
   R² and R³ each independently represent an alkyl group having 1 to 4 carbon atoms,
   R⁴ represents a hydrogen atom or an alkyl group having 1 to 4 carbon atoms,
   four of R⁵ to R⁹ each independently represent a hydrogen atom or an alkyl group having 1 to 6 carbon atoms, and one of R⁵ to R⁹ represents a bonding position with a main chain of the vinyl polymer (A), and
      * represents the bonding position with the main chain of the vinyl polymer (A).
[6] In the biocompatible polymer composition, the compound (B) has at least one structure represented by General formulas 4 to 9 below: 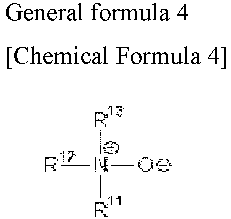 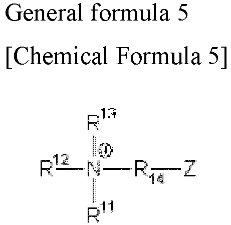 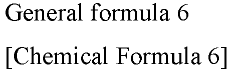 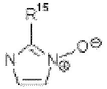 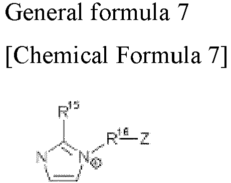 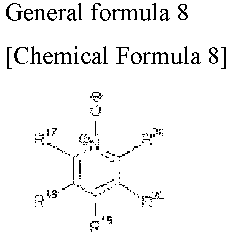 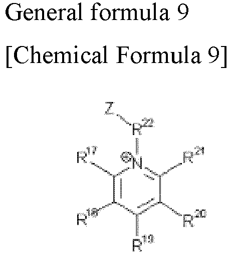 [In General formulas 4 to 9,
   R¹¹, R¹², and R¹³ each independently represent an alkyl group or a hydroxyalkyl group having 1 to 5 carbon atoms, any two of R¹¹, R¹², and R¹³ are linked to form a ring,
   R¹⁴ represents an alkylene group having 1 to 4 carbon atoms,
   Z represents -COO⁻ or SO3⁻,
   R¹⁵ represents a hydrogen atom or a methyl group,
   R¹⁶ represents an alkylene group having 1 to 4 carbon atoms,
   R¹⁷ to R²¹ represent hydrogen atoms or alkyl groups having 1 to 6 carbon atoms, and
   R²² represents an alkylene group having 1 to 4 carbon atoms.]
[7] A protein stabilizer includes the biocompatible polymer composition.
[8] A method for stabilizing a protein allows the protein stabilizer and a protein to coexist.
[9] A blocking agent includes the biocompatible polymer composition.
[10] A method for detecting a target substance in a sample through in-vitro diagnostics uses the blocking agent.
[11] A cell culture medium additive includes the biocompatible polymer composition.
[12] A cell culture medium includes the cell culture medium additive.
[13] In the cell culture medium, a concentration of the biocompatible polymer composition in the cell culture medium is 0.01 mass% or more and 1 mass% or less.
[14] A method for manufacturing a cell aggregate or a bioactive substance includes culturing cells by using the cell culture medium.

### Effects of Invention

According to the disclosure, it is possible to provide a biocompatible polymer composition having excellent handling properties that serves as a protein stabilizer having excellent protein stabilizing performance, a blocking agent having an excellent sensitizing effect, and a cell culture medium additive capable of culturing antibody-producing cells at excellent antibody productivity and cell activity

### DESCRIPTION OF EMBODIMENTS

### <Biocompatible polymer composition>

The biocompatible polymer composition according to the disclosure includes a vinyl polymer (A), which has an amine oxide group and a mass-average molecular weight of 5,000 or more, and water, and the surface tension of a 1% aqueous solution of which at 25°C is 30 dyne/cm to 50 dyne/cm. With such configuration, excellent protein stabilization effects and effectiveness as a blocking agent and a cell culture medium additive, etc., are exhibited.

### <Vinyl polymer (A)>

The biocompatible polymer composition of the disclosure includes the vinyl polymer (A), which has an amine oxide group and a mass-average molecular weight of 5,000 or more.

As the vinyl polymer (A), specifically, it is preferable to include at least one of the structures represented by the general formulas 1 to 3 described above, and the structure represented by the general formula 1 is particularly preferable. By having the amine oxide group represented by the above formula, the polymer can exhibit suitable water solubility.
The vinyl polymer (A) is preferably a copolymer, and may also be any of a block copolymer, a random copolymer, or an alternating copolymer.

The vinyl polymer (A) having an amine oxide group can be obtained, for example, by the following methods.
(1) A method of obtaining a vinyl polymer by polymerizing a tertiary amino group-containing monomer (a) and, if necessary, other monomers, and then obtaining the product by reacting the tertiary amino group with an oxidizing agent.
(2) A method of obtaining the product as a copolymer of a reaction product monomer (amine oxide group-containing monomer) of a tertiary amino group-containing monomer (a) with an oxidizing agent, and other monomers.
The amine oxide group can be introduced into the polymer or the monomer by reacting an oxidizing agent (hereinafter also referred to as an oxide agent) with the tertiary amino group. Such reaction is also referred to as "oxidation".

Among the above methods, the method of (1) is preferable in terms of being less likely to cause a side reaction. In the method of (1), it is preferable to polymerize other monomers in addition to the tertiary amino group-containing monomer (a).

### <Tertiary amino group-containing monomer (a)>

Examples of the tertiary amino group-containing monomer (a) as the precursor before oxidation, which form the structure of general formula 1, include:
N,N-dimethylaminoethyl (meth)acrylate, N,N-diethylaminoethyl (meth)acrylate, N,N-dimethylaminopropyl (meth)acrylate, N,N-diethylaminopropyl (meth)acrylate, N,N-dimethylaminoethyl (meth)acrylamide, N,N-diethylaminoethyl (meth)acrylamide, N,N-dimethylaminopropyl (meth)acrylamide, N,N-diethylaminopropyl (meth)acrylamide, vinyl N,N-dimethylaminopropionate, vinyl N,N-diethylaminopropionate, N,N-dimethylacrylamide, N,N-dimethylallylamine, p-dimethylaminomethylstyrene, p-dimethylaminoethylstyrene, p-diethylaminomethylstyrene, p-diethylaminoethylstyrene, N,N-dimethylvinylamine, N,N-diethylvinylamine, N,N-diphenylvinylamine, or reaction products of acid anhydride containing an unsaturated group, such as maleic anhydride, itaconic anhydride, citraconic anhydride, etc., with N,N-dimethyl-1,3-propanediamine, etc., reaction products of an unsaturated compound containing an epoxy group such as glycidyl (meth)acrylate, etc. with N,N-dimethyl-1,3-propanediamine, etc. The tertiary amino group-containing monomer (a) may preferably be a (methyl)acrylate monomer, and more preferably be N,N-dialkylaminoethyl (meth)acrylate or N,N-dialkylaminopropyl (meth)acrylate. In the disclosure, "(meth)acryl" refers to both "acryl" and "methacryl", and "(meth)acrylate" refers to both "acrylate" and "methacrylate".

Examples of the tertiary amino group-containing monomer (a) for forming the structure of the general formula 2 include:
1-vinylimidazole, 2-methyl-1-vinylimidazole, 4-methyl-1-vinylimidazole, 5-methyl-1-vinylimidazole, 2-lauryl-1-vinylimidazole, 4-(t-butyl)-1-vinylimidazole.

Examples of the tertiary amino group-containing monomer (a) for forming the structure of the general formula 3 include:
2-vinylpyridine, 3-vinylpyridine, 4-vinylpyridine, 2-methyl-3-vinylpyridine, 2-methyl-4-vinylpyridine, 3-methyl-4-vinylpyridine, 2-methyl-5-vinylpyridine, 3-methyl-5-vinylpyridine, 4-methyl-5-vinylpyridine, 2-lauryl-4-vinylpyridine, 2-lauryl-5-vinylpyridine, 2-(t-butyl)-4-vinylpyridine, 2-(t-butyl)-5-vinylpyridine.

### <Other monomers>

At the time of obtaining the vinyl polymer (A), other monomers having one ethylenic unsaturated group per molecule can be used in addition to the tertiary amino group-containing monomer (a). By introducing structures based on other monomers, polarity and Tg can be suitably controlled, and solvent solubility and other properties can be regulated.

Examples may include:
monomers possessing an alkyl group having 1 to 18 carbon atoms;
acrylic ester (meth)acrylates such as methoxyethyl (meth)acrylate; aromatic ester (meth)acrylates such as benzyl (meth)acrylate;
monomers having a hydroxyl group such as 2-hydroxyethyl (meth)acrylate;
monomers having a carboxylic acid group, such as maleic acid, acrylic acid, methacrylic acid, or an anhydride thereof;
monomers having primary to tertiary amide groups such as N-vinyl-2-pyrrolidone; monomers having a quaternary amino group such as (meth)acrylic acid dimethylaminoethyl methyl chloride salt, trimethyl-3-(1-(meth)acrylamide-1,1-dimethylpropyl)ammonium chloride, trimethyl-3-(1-(meth)acrylamidopropyl)ammonium chloride, and trimethyl-3-(1-(meth)acrylamide-1,1-dimethylethyl)ammonium chloride;
monomers having a polyether chain such as polyethylene glycol (meth)acrylate; (meth)acrylate-based monomers having a polymer structure on a side chain such as an ethylenically unsaturated compound having a polyester chain like lactone-modified (meth)acrylate; aromatic vinyl monomers such as styrene;
ethylenically unsaturated monomers having a nitrile group such as (meth)acrylonitrile;
fatty acid vinyl compounds such as vinyl acetate;
ethylenically unsaturated monomers of vinyl ether type such as butyl vinyl ether

From the viewpoint of introducing a hydrophobic group, it is preferable to use a monomer (b) having an alkyl group with 1 to 18 carbon atoms. In the case where the vinyl polymer (A) includes a structural unit based on the monomer (b) possessing an alkyl group with 1 to 18 carbon atoms, the content thereof is preferably 5 mass% to 70 mass%, more preferably 10 mass% to 60 mass%, and even more preferably 15 mass% to 50 mass% with respect to the total of 100 mass% of the monomer units constituting the vinyl polymer (A).

### <Oxidation>

The tertiary amino group derived from the tertiary amino group-containing monomer (a) can be converted to an amine oxide group by using various oxidizing agents. This allows for the introduction of the amine oxide group into the vinyl polymer (A). The oxidation may be performed before or after the polymerization of the vinyl polymer (A). For oxidation before polymerization, an oxidizing agent can be added to a solution containing the tertiary amino group-containing monomer (a), and for oxidation after polymerization, an oxidizing agent can be added to a solution containing the vinyl polymer polymerized from the monomer essentially including the tertiary amino group-containing monomer (a), and then, the tertiary amino group can be oxidized by reacting at a temperature range of 20°C to 100°C for 0.1 hours to 100 hours, preferably 1 hours to 50 hours.

As the oxidizing agent, an oxidizing agent such as peroxide or ozone is used. Examples of the peroxide include hydrogen peroxide, ammonium persulfate, sodium persulfate, peracetic acid, meta-chloroperoxybenzoic acid, benzoyl peroxide, and t-butyl hydroperoxide, with hydrogen peroxide being preferred. These are typically used in the form of aqueous solution.

### <Mass-average molecular weight (Mw)>

In the disclosure, and as is well known in the technical field, weight-average molecular weight and mass-average molecular weight can be used interchangeably, and both are denoted as Mw. The mass-average molecular weight of the vinyl polymer (A) is 5,000 or more, and preferably 5,000 to 500,000. By having the molecular weight within the above range, it is possible to make adjustment to a suitable surface tension when made into an aqueous solution. Furthermore, handling becomes easier from the viewpoint of defoaming properties when added to proteins or cell culture media as an aqueous solution.

The biocompatible polymer composition of the disclosure is used by being added to substances such as proteins, antibodies, and cells, therefore the interaction occurring at the interface between substances is important. In the disclosure, by adjusting the surface tension of the biocompatible polymer composition when made into a 1% aqueous solution to a suitable range, it is possible to improve protein stability, blocking properties, and the productivity of monoclonal antibodies and bioactive substances during cell culture. Although the details are not fully clear, it is considered that the suitable surface tension, or the biocompatible polymer providing the same assists in the interaction between the vinyl polymer (A) and proteins or cells.

### <Water>

The biocompatible polymer composition of the disclosure includes water in addition to the vinyl polymer (A) having the amine oxide group. The water is not particularly limited and can be, for example, sterilized purified water, pure water, ion-exchanged water, distilled water, pure water, ultrapure water, etc. Nevertheless, the water is preferably sterilized purified water. To prevent the interference with the functions of other components included in the biocompatible polymer composition, it is preferable to increase the purity of the water by using, for example, removing impurity ions using an ion exchange resin, removing foreign substances using a filter, or distillation.

In the following description, water means sterilized purified water unless otherwise specified.

### <Surface tension (dyn/cm)>

The biocompatible polymer composition of the disclosure has a surface tension in the range of 30 dyn/cm to 50 dyn/cm at 25°C when made into a 1% aqueous solution, and it is more preferable to be in the range of 30 dyn/cm to 48 dyn/cm. In the disclosure, the concentration of the aqueous solution of the biocompatible polymer composition is expressed as a percentage calculated by using the mass of the dissolved the vinyl polymer (A), or in case the composition includes a compound (B), the total mass of dissolved (A) and (B) as the numerator, and the mass of water dissolving (A) and (B) is set as the denominator. In the expression of the concentration, the water providing the denominator may, depending on the context, consist of water included in the biocompatible polymer composition product itself, or may include additional water in case the biocompatible polymer composition product is diluted with additional water. For example, in the above, when it is stated that the biocompatible polymer composition has a surface tension of 30 dyn/cm to 50 dyn/cm when made into a 1% aqueous solution, it does not necessarily mean that the biocompatible polymer composition itself is a 1% aqueous solution, but means that when the water in the biocompatible polymer composition is increased or decreased to be adjusted to a 1% aqueous solution with respect to the components (A) and (B), the surface tension is 30 dyn/cm to 50 dyn/cm.

Surface tension is a factor that affects the interface between substances and can be adjusted by adding ionic compounds such as sodium chloride and potassium chloride, non-ionic compounds such as propylene glycol and glycerin, or a surfactant. However, in an embodiment of the disclosure, the compound (B), which has an amphoteric ion structure and has a molecular weight of 500 or less, as described later, is used.

Molecules on the liquid surface receive a strong attractive force from the internal molecules. The resulting force acts to create a smallest possible liquid surface. The magnitude of such force is called surface tension and is expressed in a unit of force per unit length. In the disclosure, surface tension refers to the surface tension at a liquid temperature of 25°C. At such temperature, the surface tension of pure water is 72 dyn/cm. In other words, the biocompatible polymer composition serves to lower the surface tension.

### (Method for measuring surface tension)

Examples of methods for measuring surface tension include the Wilhelmy method (plate method, vertical plate method), the du Nouy method (ring method), the pendant drop method, the maximum bubble pressure method, and the method of calculating from Young's equation by measuring the contact angle. While any of the methods may be used, the disclosure adopts the Wilhelmy method to measure the surface tension of the biocompatible polymer composition in the state of a 1% aqueous solution at 25°C.

### <Osmotic Pressure (mOsm/kg)>

In the disclosure, it is preferable that the osmotic pressure of the biocompatible polymer composition, when made into a 1% aqueous solution, is in the range of 1 mOsm/kg to 20 mOsm/kg, and more preferably in the range of 2 mOsm/kg to 17 mOsm/kg. When the osmotic pressure is within the above range, the osmotic pressure can act synergistically with the vinyl polymer (A) and the surface tension, and further improve protein stability, blocking properties, and the productivity of monoclonal antibodies and bioactive substances during cell culture.

Osmotic pressure is proportional to the number of moles of dissolved particles divided by the volume of the solvent (L), and is expressed in the unit of Osm/L for osmolar concentration. In the case of aqueous solution, the osmolar concentration can be expressed as Osm/kg. In particular, osmotic pressure is involved in the movement of water between the inside and outside of cells and generated by solutes such as electrolytes, carbohydrates, and amino acids. In an embodiment of the disclosure, the osmotic pressure of the biocompatible polymer composition is adjusted by containing the compound (B), which will be described later. By having an osmotic pressure of 1 mOsm/kg or more when made into a 1% aqueous solution, damage to cells can be prevented. Additionally, by having an osmotic pressure of 20 mOsm/kg or less when made into a 1% aqueous solution, the risk of intracellular dehydration can be reduced.

### <Compound (B)>

In the disclosure, it is preferable that the biocompatible polymer composition further includes the compound (B), which possesses an amphoteric ion structure and has a molecular weight of 500 or less. However, the compound (B) excludes surfactants and monomers (specifically, the monomers used to produce the vinyl polymer (A)). Nevertheless, as long as it does not deviate from the essence of the disclosure, it does not prevent unintended inclusion of residual monomers and the like. In the disclosure, the amount of the compound (B) is preferably 0.5 to 10 parts per 100 parts of the vinyl polymer (A), and more preferably 1 to 5 parts. In an embodiment, the biocompatible polymer composition does not include a surfactant. In an embodiment, the biocompatible polymer composition consists of the vinyl polymer (A), the compound (B), and water.

The compound (B) preferably includes at least one structure represented by any of the general formulas 4 to 9 described above, and among the compounds, those including structures represented by the general formula 4 and/or 5 are particularly preferable. With the compound (B) represented by the above formulas, it becomes easier to achieve a suitable surface tension for the biocompatible polymer composition.

The compound (B) can be synthesized, for example, by using a compound having a tertiary amino group as a starting material.

For example, to obtain a compound having an amine oxide structure, an oxide agent such as peroxide or ozone is used on a compound having a tertiary amino group.

Additionally, for example, to obtain a compound having a carboxybetaine structure, cyclic carboxylic acid esters such as β-propiolactone, γ-butyrolactone, δ-valerolactone, or ω-halogenated alkyl carboxylic acid metal salts such as sodium 2-chloroacetate, sodium 2-bromoacetate, sodium 3-chloropropionate, sodium 3-bromopropionate, sodium 4-chlorobutyrate, sodium 4-bromobutyrate, sodium 5-chloropentanoate, sodium 5-bromopentanoate are used in a compound having a tertiary amino group.

Furthermore, for example, to obtain a compound having a sulfobetaine structure, cyclic sulfonic acid esters such as 1,2-ethanesultone, 1,3-propanesultone, 1,4-butanesultone, or ω-halogenated alkyl sulfonic acid metal salts such as sodium 2-chloroethanesulfonate, sodium 2-bromoethanesulfonate, sodium 3-chloropropanesulfonate, sodium 3-bromopropanesulfonate, sodium 4-chlorobutanesulfonate, sodium 4-bromobutanesulfonate are used in a compound having a tertiary amino group.

As a compound for forming the structure of the general formula 4 or 5, examples may include:
trimethylamine N-oxide, dimethyl-2-hydroxyethylamine N-oxide, N,N,N-trimethylglycine, dimethylethylammonium propanesulfonic acid, dimethyl-2-hydroxyethylammonium propanesulfonic acid, dimethylbenzylammonium propanesulfonic acid, and 3-(1-methylpiperidinium)-1-propanesulfonic acid.

As a compound for forming the structure of the general formula 6 or 7, examples may include:
imidazole N-oxide, 2-methyl-imidazole N-oxide, imidazolinium betaine, and 3-(3-sulfopropyl)imidazolium inner salt.

As a compound for forming the structure of the general formula 8 or 9, examples may include:
pyridine N-oxide, 1-(3-sulfopropyl)pyridinium inner salt, and 1-(4-sulfobutyl)pyridinium inner salt.

The following describes in detail various applications including protein stabilizers, blocking agents, and cell culture medium additives.

### <Protein stabilizer>

The biocompatible polymer composition of the disclosure can stably store protein by coexisting with the protein. The stabilizer and the protein can coexist by being dissolved or dispersed by a common solvent. The solvent adopted for the stabilizer and protein to co-exist is preferably an aqueous solvent. Such solvent includes, for example, water, tris-buffered saline (TBS), and phosphate-buffered saline (PBS). The water may partially include an organic solvent that can be mixed with water, such as methanol, ethanol, propyl alcohol, or tetrahydrofuran. The stabilizer of the disclosure is preferably used by adding 100 times, more preferably 10,000 times by mass to the protein. In the case where the amount of stabilizer added is insufficient, it is possible that the stabilizing effect cannot be exhibited. For the same reason, the stabilizer, i.e., the biocompatible polymer composition, is preferably used by adding 0.01% or more, more preferably 0.1% or more to the total mass of the protein solution.

### <Blocking agent>

The biocompatible polymer composition of the disclosure can be used as a blocking agent. The blocking agent is used to prevent non-specific adsorption of antibody proteins to sites where specific antigens are not present, and to enhance specific antigen-antibody reactions. The blocking agent can be used, for example, in in-vitro diagnostics to detect a target substance in samples. When using the biocompatible polymer composition as a blocking agent, the concentration thereof is preferably 0.01% by mass or more and 5% by mass or less, more preferably 0.01% by mass or more and 1% by mass or less. If the concentration of the biocompatible polymer composition is low, the effect on improving antigen-antibody reaction efficiency or reducing non-specific reactions is small, and if the concentration is high, the antigen-antibody reaction may be obstructed. The blocking agent referred to herein is used to pretreat samples containing antigens before antibody reactions to prevent non-specific adsorption of antibodies to sites where specific antigens are not present, or to be added to a dilution solution for antibody reaction to prevent non-specific antibody reaction, and the form of the blocking agent is not particularly limited.

In either case, the effect can be determined by adding the blocking agent and antibody to a suitable buffer solution and comparing the degree of non-specific reaction when the composition is applied to an experimental technique utilizing immune reaction. More preferably, in enzyme immunoassay (EIA), a solution in which the blocking agent is diluted with a commonly used buffer solution such as TBS or PBS can be applied to samples containing antigens before antibody reaction, incubated at, for example, 37°C for one hour, and then the antibody reaction can be performed. This allows quantitative measurement of the degree of non-specific adsorption of antibodies to non-antigens.

### <Cell culture medium additive>

The biocompatible polymer composition of the disclosure can be used as a cell culture medium additive. Conventional cell culture media can be used. For example, various commercially available media (αMEM, MEM, DMEM, IMDEM, RPMI1640, DMEM/F 12, etc.) or combinations thereof can be mentioned. The concentration of the vinyl polymer (A) or biocompatible polymer composition, which is a cell culture medium additive, in the medium is preferably 0.001 % by mass to 1% by mass, more preferably 0.01 % by mass to 1% by mass. 0.1 % by mass to 1% by mass is even more preferable. It is preferable to add one or more of various growth factors (epidermal growth factor, insulin-like growth factor, nerve growth factor, hepatocyte growth factor, vascular endothelial growth factor, basic fibroblast growth factor, etc.), transferrin, steroid hormones, 2-mercaptoethanol, various animal sera (fetal bovine serum (FBS), bovine serum, etc.), serum substitutes, etc., to the cell culture medium as needed.

### <Method for manufacturing bioactive substance or spheroid>

By culturing cells in a cell culture medium containing the biocompatible polymer composition of the disclosure, bioactive substances produced by the cells and secreted depending on the situation can be produced with high efficiency. Additionally, by culturing cells in a cell culture medium containing the biocompatible polymer composition of the disclosure, spheroids, i.e., cell aggregates, can be efficiently formed. Cell types capable of forming spheroids are known to those skilled in the art. While methods for forming spheroids by culturing such types of cells in, for example, round-bottom wells are known, maximum formation efficiency is not always achieved in general. The cell culture medium additive of the disclosure can improve such suboptimal spheroid formation efficiency.

### <Bioactive substance>

The bioactive substances according to the embodiment include, for example, antibody proteins (hereinafter referred to as antibodies), albumin, drug-metabolizing enzymes, and the like. The antibodies produced herein are not particularly limited, and may include, for example, mouse monoclonal antibodies, humanized monoclonal antibodies, or human monoclonal antibodies. Although the class of immunoglobulin is not particularly limited, it is, for example, IgG (e.g., IgG1, IgG2, etc.). Additionally, the drug-metabolizing enzymes that are produced are not particularly limited. Examples may include, for example, alcohol dehydrogenase, aldehyde dehydrogenase, glutathione peroxidase, superoxide dismutase, monoamine oxidase, diamine oxidase, epoxide hydrolase, esterase, amidase, UDP-glucuronosyltransferase, glutathione S-transferase, γ-glutamyl transpeptidase, acetyltransferase, sulfotransferase, cytochrome P450, and the like.

In the culture method or manufacturing method of the disclosure, containers or devices commonly used for culture can be used when culturing. Examples may include multi-well plates, petri dishes, culture flasks, spinner flasks, jar fermenters, fermenters, roller bottles, hollow fibers, and microcarriers.

### <Cell>

The cells cultured by using the culture method of the disclosure are not particularly limited as long as as the cells are capable of producing bioactive substances. For example, cells capable of producing antibodies include CHO cells, BHK cells, HepG2 cells, rodent myeloma cells (such as mouse myeloma cells like SP2/O cells, NSO cells, etc.), hybridomas, insect cells, and transformed cells into which foreign genes are introduced. For instance, hybridomas obtained by cell fusion of CHO cells, SP2/O cells, or NSO cells can be adopted. As cells capable of producing drug-metabolizing enzymes, examples include primary hepatocytes obtained from animal livers, hepatocytes differentiated from stem cells such as ES cells, iPS cells, and mesenchymal stem cells, as well as liver cancer-derived cell lines such as HepG2 cells and HuH-7 cells.

The culture conditions in the embodiment can be the usual conditions for animal cell culturing, and can be set, for example, to a temperature of 37°C under a 5 vol% CO₂ atmosphere.

To collect cells from the culture solution, in the case of suspension cells, for example, the culture solution can be directly subjected to a centrifugal separator or a filter to gather the cells. In the case of adherent cells, for example, 0.25% trypsin-0.02% EDTA solution is added to suspend the cells, and then the suspended cells are collected through centrifugal separation or filtering.

Bioactive substances, especially antibodies, produced through cell culturing are collected from the supernatant obtained by filtering or centrifugal separation, in the case where the bioactive substances accumulate in the culture solution. In the case where substances accumulate intracellularly, the cells obtained by filtering or centrifugal separation are subjected to homogenization, ultrasonic treatment, chemical treatment, etc., to obtain a supernatant in which products are extracted.

The separation and/or purification of antibodies from the supernatant can be performed by suitably combining conventional methods. For example, ammonium sulfate precipitation, dialysis, ultrafiltration, electrophoresis, gel filtration, ion exchange chromatography, reverse phase chromatography, and affinity chromatography are preferable.

### Example

The following examples more specifically describe embodiments of the disclosure, but these examples do not in any way limit the scope of rights of the disclosure. In the examples and comparative examples, "parts" and "%" represent "parts by mass" and "mass%", respectively, "mol" represents the amount of substance, and "mol%" represents the proportion of the amount of substance of the target monomer in the total monomers.

### (Method for measuring mass-average molecular weight (Mw))

The mass-average molecular weight was adopted as the value measured by gel permeation chromatography (GPC) through standard pullulan conversion. The measurement apparatus and measurement conditions as follows in Condition 1 were used. For other matters, JISK7252-1 to 4:2008 was used as reference. For poorly soluble polymer compounds, measurements were made at concentrations that allowed dissolution under the following conditions. In the case where it is difficult to measure the molecular weight of the vinyl polymer (A), the mass-average molecular weight of the amine oxide precursor polymer can be used as the mass-average molecular weight of the vinyl polymer (A). Regarding the mass-average molecular weight of the amine oxide precursor polymer, the value measured by gel permeation chromatography (GPC) through standard polystyrene conversion is used, and the measurement apparatus and conditions generally follows Condition 2 below.

### (Condition 1)

Column: OHpak SB-G, three OHpak SB-806M HQ, and OHpak SB-802.5 HQ connected in series.
Carrier: 1/15 mol/L pH 7.0 phosphate buffer (prepared by dissolving phosphate buffer powder 1/15 mol/L pH 7.0 (manufactured by FUJIFILM Wako Pure Chemical Corporation) in 1 L of ion-exchanged water)
Carrier flow rate: 1.0 mL/min
Sample concentration: 0.5 mass%
Detector: RI (refractive index) detector
Injection volume: 0.1 mL

### (Condition 2)

Column: TOSOH TSKgel Super AW4000, TOSOH TSKgel Super AW3000, and TOSOH TSKgel
Super AW2500 connected in series.
Carrier: A mixed solution of N,N-dimethylformamide (1 L), triethylamine (3.04 g), and LiBr (0.87 g)
Measurement temperature: 40°C
Carrier flow rate: 0.6 mL/min

### (Method for measuring surface tension)

The biocompatible polymer composition was prepared as a 1% aqueous solution, and the surface tension was measured at 25°C using the Wilhelmy method.

### (Method for measuring osmotic pressure)

The method for measuring the osmotic pressure followed the method for measuring the osmotic pressure described in the 16th edition of the Japanese Pharmacopoeia. The osmolar concentration of the biocompatible polymer composition was measured as a 1% aqueous solution by measuring the freezing point decrease degree of water.

### <Manufacture of vinyl polymer (A)>

### [Manufacture Example 1]

### (P-1)

In a reaction vessel including a thermometer, a stirrer, a nitrogen introduction tube, a reflux cooler, and a dropping funnel, 150 parts by mass of ethyl acetate as an organic solvent was charged under a nitrogen gas flow, and heated at 80°C for 30 minutes with stirring. 70 parts by mass of N,N-dimethylaminoethyl methacrylate and 30 parts by mass of butyl methacrylate as monomers, 0.3 parts by mass of azobisisobutyronitrile (AIBN) as a polymerization initiator, and 10 parts by mass of ethyl acetate as a solvent were charged to the dropping funnel and added dropwise over 2 hours. After completion of the dropwise addition, heating was continued for 6 hours. Subsequently, the product was cooled to room temperature and the reaction was stopped.

Next, 150 parts of ethanol and 43 parts of 35% hydrogen peroxide water as an oxidizing agent (equimolar amount to the N,N-dimethylaminoethyl methacrylate that was used) were added, and the reaction was carried out at 70°C for 16 hours to oxidize the tertiary amino group. Subsequently, the solvent was removed by using a diaphragm pump to obtain a vinyl polymer possessing an amine oxide group.

### [Manufacture Examples 2 to 4, and Comparative Manufacture Example 1]

### (P-2 to P-5)

Vinyl polymers were obtained in the same manner as P-1, except that the compositions were changed as shown in Table 1.

**[Table 1]**

| | | Monomer (a) | | | Monomer (b) | | | Monomer (c) | AIBN | 35% Hydrogen peroxide water | Mass-average molecular weight |
|---|---|---|---|---|---|---|---|---|---|---|---|
| | | DEAEMA | VP | VI | ISTA | BMA | MMA | St | | | |
| Manufacture Example 1 | P-1 | 70 | | | | 30 | | | 0.3 | 43 | 32000 |
| Manufacture Example 2 | P-2 | 80 | | | 20 | | | | 0.3 | 49 | 18400 |
| Manufacture Example 3 | P-3 | | 70 | | | | 30 | | 0.5 | 65 | 5300 |
| Manufacture Example 4 | P-4 | | | 70 | | | | 30 | 0.5 | 72 | 7500 |
| Comparative Manufacture Example 1 | P-5 | 70 | | | | 30 | | | 15 | 43 | 1800 |

The details of the abbreviations in Table 1 are as follows.
DEAEMA: N,N-diethylaminoethyl methacrylate
VP: vinylpyridine
VI: vinylimidazole
ISTA: isostearyl acrylate
BMA: butyl methacrylate
MMA: methyl methacrylate
St: styrene

### <Manufacture of biocompatible polymer composition>

### [Example 1-1]

### (Additive 1)

The vinyl polymer (P-1) obtained above and trimethylglycine (TMG) were dissolved in water, so that TMG was 2 parts with respect to 100 parts of P-1, and a biocompatible polymer composition with a concentration of 1% by mass was obtained. The surface tension was 43 dyn/cm, and the osmotic pressure was 2 mOsm/kg. The result of the defoaming evaluation, which was assessed as an indicator of handling, was ∘ (good).

### [Examples 1-2 to 1-22 and Comparative Examples 1-1 to 1-4]

### (Additives 2-26)

Preparation same as Example 1-1 was performed except for the changes to the compositions indicated in Table 2.

### [Defoaming property evaluation]

Biocompatible polymer compositions are generally stirred before use. However, if bubbles are generated during stirring, inconvenience is caused from the perspective of handling. Therefore, the defoaming properties of bubbles generated after stirring were evaluated as an indicator of the handling properties by using the following method.

3 mL of a 1% aqueous solution of the biocompatible polymer composition was added to a 15 mL centrifuge tube, vigorously shaken by hand, and then left to stand for 30 minutes. The height of the foam after standing was measured and evaluated according to the following criteria.

### Height of foam after 30 minutes of standing

∘: Less than 3 cm
×: 3 cm or more

**[Table 2]**

| Table 2 | | Vinyl polymer (A) | | Compound (B) | | | | | | | Surface tension dyn/cm | Osmotic pressure mOsm/kg | Defoaming properties |
|---|---|---|---|---|---|---|---|---|---|---|---|---|---|
| | | | Preparation amount | TMG | NDSB195 | NDSB201 | NDSB211 | TMAO | DMHEAO | PyO | | | |
| Example 1-1 | Additive 1 | P-1 | 100 | 2 | | | | | | | 43 | 2 | ○ |
| Example 1-2 | Additive 2 | P-1 | 100 | | 0.5 | | | | | | 45 | 1 | ○ |
| Example 1-3 | Additive 3 | P-1 | 100 | | | 5 | | | | | 38 | 3 | ○ |
| Example 1-4 | Additive 4 | P-1 | 100 | | | | 10 | | | | 35 | 5 | ○ |
| Example 1-5 | Additive 5 | P-1 | 100 | | | | | 0.5 | | | 46 | 1 | ○ |
| Example 1-6 | Additive 6 | P-1 | 100 | | | | | | 3 | | 40 | 3 | ○ |
| Example 1-7 | Additive 7 | P-1 | 100 | | | | | | | 10 | 34 | 10 | ○ |
| Example 1-8 | Additive 8 | P-1 | 100 | | | | 2 | | 2 | | 49 | 3 | ○ |
| Example 1-9 | Additive 9 | P-2 | 100 | 0.5 | | | | | | | 45 | 1 | ○ |
| Example 1-10 | Additive 10 | P-2 | 100 | | 10 | | | | | | 32 | 5 | ○ |
| Example 1-11 | Additive 11 | P-2 | 100 | | | | | 3 | | | 40 | 4 | ○ |
| Example 1-12 | Additive 12 | P-2 | 100 | | | | | | 3 | | 40 | 3 | ○ |
| Example 1-13 | Additive 13 | P-3 | 100 | 1 | | | | | | | 44 | 3 | ○ |
| Example 1-14 | Additive 14 | P-3 | 100 | | | 0.5 | | | | | 48 | 2 | ○ |
| Example 1-15 | Additive 15 | P-3 | 100 | | | | | | 10 | | 34 | 10 | ○ |
| Example 1-16 | Additive 16 | P-4 | 100 | 10 | | | | | | | 30 | 9 | ○ |
| Example 1-17 | Additive 17 | P-4 | 100 | | | | 2 | | | | 40 | 2 | ○ |
| Example 1-18 | Additive 18 | P-4 | 100 | | | | | | | 0.5 | 47 | 2 | ○ |
| Example 1-19 | Additive 19 | P-2 | 100 | 0.4 | | | | | | | 48 | 1 | ○ |
| Example 1-20 | Additive 20 | P-3 | 100 | | | | 15 | | | | 31 | 8 | ○ |
| Example 1-21 | Additive 21 | P-4 | 100 | | 10 | | | 5 | | | 30 | 12 | ○ |
| Example 1-22 | Additive 22 | P-1 | 100 | | | | | | 20 | | 30 | 17 | ○ |
| Comparative Example 1-1 | Additive 23 | P-1 | 100 | | | | | | | | 55 | 0 | × |
| Comparative Example 1-2 | Additive 24 | P-5 | 100 | | | | | | | | 52 | 5 | × |
| Comparative Example 1-3 | Additive 25 | P-5 | 100 | | | 5 | | | | | 51 | 7 | × |
| Comparative Example 1-4 | Additive 26 | P-5 | 100 | | | | 50 | | | | 30 | 22 | × |

The details of the abbreviations in Table 2 are as follows.
TMG: Trimethylglycine
NDSB195: Dimethylethylammonium propanesulfonate
NDSB201: 3-(1-Pyridinio)propanesulfonate
NDSB211: Dimethyl-2-hydroxyethylammonium propanesulfonate
TMAO: Trimethylamine N-oxide
DMHEAO: Dimethyl-2-hydroxyethylamine N-oxide
PyO: Pyridine N-oxide

### <Protein stability test on biocompatible polymer composition>

### [Examples 2-1 to 2-22][Comparative Examples 2-1 to 2-4]

Each compound was dissolved in PBS at the composition ratio shown in Table 2, and the solid content concentration was set to 0.1mass%.

### <Evaluation of protein stabilizer>

The following evaluation was performed on the obtained protein stabilizer. The results are shown in Table 3.

### [Evaluation on preparation of enzyme aqueous solution]

HRP-labeled CRP antibody (manufactured by abcam) was diluted and mixed with 1 ml of the obtained protein stabilizer aqueous solution, so that the HRP-labeled CRP antibody became 8.0 ng/ml, and an enzyme aqueous solution for evaluation was prepared. The enzyme aqueous solution for evaluation was tightly sealed and stored at 40°C, so as not to change the concentration.

### [Enzyme activity evaluation]

By using a peroxidase color development kit (manufactured by Sumitomo Bakelite), a color development solution was obtained by mixing 10 ml of a color development agent (3,3',5,5'-tetramethylbenzidine (TMBZ) solution) and 100 µl of a substrate solution (hydrogen peroxide solution).

100 µl of the color development solution and 100 µl of the enzyme aqueous solution for evaluation were added to a 96-well plate and left in a dark place at 30°C for 20 minutes. 100 µl of a stop solution (sulfuric acid aqueous solution) was added to stop the reaction. The absorbance at 450 nm was measured by using a plate reader Multimode Reader Mithras2 LB943 (manufactured by BERTHOLD TECHNOLOGIES), and the amount of TMBZ oxide produced through the reaction was quantified. Since the oxidation reaction of TMBZ correlates with the activity of the HRP-labeled CRP antibody, the obtained absorbance can be used as an indicator of enzyme activity.

### <Evaluation of protein stabilizing effect>

The enzyme activity evaluation was performed twice, i.e., before storage of the enzyme aqueous solution for evaluation and three days after storage at 40°C, and the absorbance was measured. The ratio of the absorbance after storage when the absorbance before storage was set as 100% was calculated as the relative absorbance, and the denaturation of protein was evaluated according to the following criteria.

### Relative absorbance at 40°C and after storage for 3 days

Ⓞ: 70% or higher (Extremely good)
○: 50% or higher and less than 70% (Good)
△: 30% or higher and less than 50% (Usable)
×: Less than 30% (Unusable)

**[Table 3]**

| Table 3 | | Absorbance | | Protein stabilization effect | |
|---|---|---|---|---|---|
| | | before storage | After storage at 40°C for 3 days | relative absorbance | determination |
| Example 2-1 | Composition 1 | 2.10 | 1.53 | 73 | Ⓞ |
| Example 2-2 | Composition 2 | 2.09 | 1.48 | 71 | Ⓞ |
| Example 2-3 | Composition 3 | 2.08 | 1.48 | 71 | Ⓞ |
| Example 2-4 | Composition 4 | 2.09 | 1.55 | 74 | Ⓞ |
| Example 2-5 | Composition 5 | 2.09 | 1.67 | 80 | Ⓞ |
| Example 2-6 | Composition 6 | 2.10 | 1.72 | 82 | Ⓞ |
| Example 2-7 | Composition 7 | 2.08 | 1.48 | 71 | Ⓞ |
| Example 2-8 | Composition 8 | 2.10 | 1.70 | 81 | Ⓞ |
| Example 2-9 | Composition 9 | 2.09 | 1.57 | 75 | Ⓞ |
| Example 2-10 | Composition 10 | 2.10 | 1.53 | 73 | Ⓞ |
| Example 2-11 | Composition 11 | 2.09 | 1.57 | 75 | Ⓞ |
| Example 2-12 | Composition 12 | 2.08 | 1.64 | 79 | Ⓞ |
| Example 2-13 | Composition 13 | 2.10 | 1.49 | 71 | Ⓞ |
| Example 2-14 | Composition 14 | 2.08 | 1.50 | 72 | Ⓞ |
| Example 2-15 | Composition 15 | 2.09 | 1.55 | 74 | Ⓞ |
| Example 2-16 | Composition 16 | 2.10 | 1.49 | 71 | Ⓞ |
| Example 2-17 | Composition 17 | 2.10 | 1.58 | 75 | Ⓞ |
| Example 2-18 | Composition 18 | 2.08 | 1.48 | 71 | Ⓞ |
| Example 2-19 | Composition 19 | 2.09 | 0.92 | 44 | △ |
| Example 2-20 | Composition 20 | 2.10 | 1.34 | 64 | ○ |
| Example 2-21 | Composition 21 | 2.10 | 1.11 | 53 | ○ |
| Example 2-22 | Composition 22 | 2.10 | 1.24 | 59 | ○ |
| Comparative Example 2-1 | Composition 24 | 2.08 | 0.60 | 29 | × |
| Comparative Example 2-2 | Composition 25 | 2.08 | 0.31 | 15 | × |
| Comparative Example 2-3 | Composition 26 | 2.10 | 0.15 | 7 | × |
| Comparative Example 2-4 | Composition 27 | 2.05 | 0.35 | 17 | × |

As shown in Table 3, by using the biocompatible polymer composition of the disclosure as a protein stabilization agent, a high protein stabilizing effect was obtained. Meanwhile, Comparative Examples 2-1 to 2-4, which did not have the vinyl polymer (A) or had a surface tension of the aqueous solution outside the range specified in the disclosure, did not exhibit a sufficient stabilizing effects even when stored in coexistence with proteins.

### <Evaluation on sensitizing effect of biocompatible polymer composition as blocking agent>

### [Examples 3-1 to 3-22][Comparative Examples 3-1 to 3-4]

Each compound was dissolved in PBS at the composition ratio shown in Table 2, and the solid content concentration was set to 1 mass%. The sensitizing effect evaluation was performed as described below, and the evaluation was based on the difference in absorbance. The results are shown in Table 4.

### [Antigen-antibody reaction test]

### (Solid-phase antibody treatment of Well Plate)

Anti-HSA antibody was adjusted with a PBS solution to a concentration of 3 µg/ml as the solid-phase antibody. 100 µl of the solid-phase antibody solution was added successively to a 96-well plate made of polystyrene, and after being left in a static state at 4°C overnight, the solution was removed by aspiration. Subsequently, 200 µl of PBS-T solution was added successively and removed by aspiration, and the process was repeated 3 times.

### <Blocking process>

200 µl of the blocking agent prepared as described above was added successively to the 96-well plate having undergone the antibody treatment, and after being left in a static state at room temperature for 2 hours, the solution was removed by aspiration. Subsequently, 200 µl of PBS-T solution was added successively and removed by aspiration, and the process was repeated 3 times.

### (Antigen adsorption treatment)

As the antigen, HSA was adjusted to the concentrations of 0 ng/ml, 25 ng/ml, 50 ng/ml, and 100 ng/ml in the PBS solution. 100 µl of the HSA antigen solution was added successively to the 96-well plate that had undergone the solid-phase antibody treatment (and blocking treatment) as described above. After being left in a static state at room temperature for 1 hour, the solution was removed by aspiration. Subsequently, 200 µl of PBS-T solution was added successively and removed by aspiration, and the process was repeated 3 times.

### (Labeled antibody adsorption treatment)

As the HRP-labeled antibody, A80-129P (anti-human albumin antibody) was adjusted by using the PBS solution to become 20 ng/ml. 100 µl of the HRP-labeled HSA antibody solution was added successively in the 96-well plate that had undergone the blocking treatment (and antigen adsorption treatment) as described above. After being left in a static state at room temperature for 1 hour, the solution was removed by aspiration. Subsequently, 200 µl of PBS-T solution was added successively and removed by aspiration, and the process was repeated 3 times.

### (Color development reaction) 100 µl of ABTS solution was added successively to the 96-well plate that had undergone the labeled antibody adsorption treatment. After being left in a static state at room temperature for 1 hour, 100 µl of stop solution was added successively.

### (Absorbance measurement)

The absorbance at 405 nm when the HSA antigen concentration was 100 ng/ml (designated as Absorbance A) and the absorbance at 405 nm when the HSA antigen concentration was 0 ng/ml (designated as Absorbance B) were measured. The sensitizing effect when using the biocompatible polymer composition as a blocking agent in ELISA was evaluated according to the following criteria.

### [Evaluation criteria]

Ⓞ: 1.5 ≤ (Absorbance A - Absorbance B): Excellent
○: 0.8 < (Absorbance A - Absorbance B) < 1.5: Usable
×: (Absorbance A - Absorbance B) ≤ 0.8: Not usable

**[Table 4]**

| Table 4 | | Sensitizing effect |
|---|---|---|
| Example 3-1 | Blocking agent composition 1 | Ⓞ |
| Example 3-2 | Blocking agent composition 2 | Ⓞ |
| Example 3-3 | Blocking agent composition 3 | Ⓞ |
| Example 3-4 | Blocking agent composition 4 | Ⓞ |
| Example 3-5 | Blocking agent composition 5 | Ⓞ |
| Example 3-6 | Blocking agent composition 6 | Ⓞ |
| Example 3-7 | Blocking agent composition 7 | Ⓞ |
| Example 3-8 | Blocking agent composition 8 | Ⓞ |
| Example 3-9 | Blocking agent composition 9 | Ⓞ |
| Example 3-10 | Blocking agent composition 10 | Ⓞ |
| Example 3-11 | Blocking agent composition 11 | Ⓞ |
| Example 3-12 | Blocking agent composition 12 | Ⓞ |
| Example 3-13 | Blocking agent composition 13 | Ⓞ |
| Example 3-14 | Blocking agent composition 14 | Ⓞ |
| Example 3-15 | Blocking agent composition 15 | Ⓞ |
| Example 3-16 | Blocking agent composition 16 | Ⓞ |
| Example 3-17 | Blocking agent composition 17 | Ⓞ |
| Example 3-18 | Blocking agent composition 18 | Ⓞ |
| Example 3-19 | Blocking agent composition 19 | ○ |
| Example 3-20 | Blocking agent composition 20 | ○ |
| Example 3-21 | Blocking agent composition 21 | ○ |
| Example 3-22 | Blocking agent composition 22 | Ⓞ |
| Comparative Example 3-1 | Blocking agent composition 24 | × |
| Comparative Example 3-2 | Blocking agent composition 25 | × |
| Comparative Example 3-3 | Blocking agent composition 26 | × |
| Comparative Example 3-4 | Blocking agent composition 27 | × |

From the results in Table 4, by using the biocompatible polymer composition of the disclosure as a blocking agent, a sensitizing effect during ELISA testing is achieved. Meanwhile, Comparative Examples 3-1 to 3-4, which either did not have the vinyl polymer (A) or had a surface tension of the aqueous solution outside the range specified in the disclosure, did not obtain a sufficient sensitizing effect as a blocking agent.

### <Cell culture test of biocompatible polymer composition>

### [Examples 4-1 to 4-22][Comparative Examples 4-1 to 4-4]

Each compound was dissolved in a cell culture medium at the composition ratios shown in Table 2, and the following evaluations were performed. The results are shown in Table 5. The evaluations of (1) the amount of production of the bioactive substance and (2) the evaluation of cell activity described below were conducted by adding a predetermined amount of the cell culture medium additive to each base medium, adjusting the medium compositions by pipetting, and evaluating the final concentrations from the range of 0.01 mass% to 1 mass%.

### (1) Evaluation of production amount of bioactive substance

### <Evaluation of antibody productivity>

Dynamis Medium (manufactured by Gibco) was used as the medium, and L-Glutamin was added to become 1.0 mass%. This is referred to as the base medium hereafter. Two sets were prepared, and the cell culture medium additive containing the obtained biocompatible polymer composition was added to achieve the concentration listed in Table 5. CHO cell lines, which were introduced with IgG genes and secreted IgG antibodies, were added to the two types of media with different concentrations of the cell medium additive containing the biocompatible polymer composition, respectively, to prepare cell suspensions with a CHO cell line concentration of 1,000,000 cells/mL. 20 mL of the obtained cell suspension was inoculated into a 125 mL Erlenmeyer flask and cultured at 37°C. During culturing, before the depletion of nutrients, 15 mL of supernatant was collected once every 3 to 4 days and replaced with 15 mL of fresh base medium, and the process was repeated 5 times. After the medium exchange was repeated 5 times, the cell culture solution was centrifuged to collect the medium supernatant. The amount of antibody in the medium was measured using the Human IgG ELISA Quantitation Set manufactured by Bethyl Laboratories, Inc. In addition, the DNA amount of the precipitated cell group was also quantified, and the antibody production amount per unit DNA amount was calculated. The IgG antibody productivity was evaluated as a relative value, with the performance in the case of culturing without addition of the cell culture medium additive containing the biocompatible polymer composition being set as 1. The evaluation results are shown in Table 5.
Ⓞ: 1.3 or higher (Very good)
○: 1 or higher to less than 1.3 (Good)
×: Less than 1 (Poor)

### <Evaluation of albumin productivity>

The medium composition was prepared by adding the cell culture medium additive containing the biocompatible polymer composition to 10% FBS-DMEM medium to achieve the concentration listed in Table 5, and then stirring. HepG2 cells, a human liver cancer cell line, were seeded to achieve 250,000 cells/mL in the medium composition containing the cell culture medium additive with the biocompatible polymer composition, and then dispensed into the wells of a 96-well U-bottom microplate at 200 µL per well. Then, the plate was cultured in a static state in a CO₂ incubator (37°C, 5% CO₂) for 3 days. After 3 days of culturing, the culture solution containing liver cells was collected and centrifuged to recover the medium supernatant. The amount of albumin in the medium was measured by using the Albumin ELISA Quantitation Set manufactured by Bethyl Laboratories, Inc. In addition, the DNA amount of the precipitated cell group was also quantified, and the albumin production amount per unit DNA amount was calculated. The albumin productivity was evaluated as a relative value, with the performance in the case of culturing without addition of the cell culture medium additive containing the biocompatible polymer composition being set as 1. The evaluation results are shown in Table 5.
Ⓞ: 1.2 or higher (Very good)
○: 1 or higher to less than 1.2 (Good)
×: Less than 1 (Poor)

### <Evaluation of drug-metabolizing enzyme activity>

The medium composition was prepared by adding the cell culture medium additive containing the biocompatible polymer composition to 10% FBS-DMEM medium to achieve the concentration listed in Table 5, and then stirring. HepG2 cells, a human liver cancer cell line, were seeded to achieve 250,000 cells/mL in the medium composition containing the cell culture medium additive with the biocompatible polymer composition, and then dispensed into the wells of a 96-well U-bottom microplate at 200 µL per well. Then, the plate was cultured in a static state in a CO₂ incubator (37°C, 5% CO₂) for 3 days. After 3 days of culturing, the drug-metabolizing enzyme activity was evaluated. The drug-metabolizing enzyme activity was assessed by using P450-GloTM CYP3A4 Assay Kits (manufactured by Promega), with Luciferin-IPA used as the substrate. The substrate was replaced with 10% FBS-DMEM medium containing the substrate and incubated for 4 hours. After incubation, the medium was transferred to a black plate for luminescence measurement, and the detection reagent (Luciferin detection reagent) was added and placed at room temperature for 20 minutes. The luminescence of the plate was measured by using a plate reader (manufactured by Berthold). In addition, the DNA amount of the cell group was also quantified, and the drug-metabolizing enzyme activity value per unit DNA amount was calculated. The drug-metabolizing enzyme activity was evaluated as a relative value, with the performance in the case of culturing without addition of the cell culture medium additive containing the biocompatible polymer composition being set as 1. The evaluation results are shown in Table 5.
Ⓞ: 1.2 or higher (Very good)
○: 1.0 or higher to less than 1.2 (Good)
×: Less than 1 (Poor)

### <Gene expression analysis by qRT-PCR Method>

The medium composition was prepared by adding the cell culture medium additive containing the biocompatible polymer composition to 10% FBS-DMEM medium to achieve the concentration listed in Table 5, and then stirring. HepG2 cells, a human liver cancer cell line, were seeded to achieve 250,000 cells/mL in the medium composition containing the cell culture medium additive with the biocompatible polymer composition, and then dispensed into the wells of a 96-well U-bottom microplate at 200 µL per well. Then, the plate was cultured in a static state in a CO₂ incubator (37°C, 5% CO₂) for 3 days. After 3 days of culturing, the culture solution containing liver cells was collected and centrifuged to recover the cells. The total RNA was extracted from the recovered HepG2 cells by using the RNeasy Micro Kit (manufactured by QIAGEN). cDNA was synthesized through the reverse transcription reaction using ReverTra Ace qPCR RT Master Mix (manufactured by TOYOBO) from the extracted total RNA. qRT-PCR was performed by using cDNA and a TaqMan probe (manufactured by LifeTechnologies, CYP3A4: Hs00604506_m1, Albumin: Hs00609411_m1) for the target genes (CYP3A4 and Albumin). GAPDH (manufactured by LifeTechnologies, Hs02786624_g1) was used as the reference gene. The relative expression level of each gene was calculated by using the comparative Ct method with GAPDH as the standard. Gene expression was evaluated as a relative value, with the performance in the case of culturing without addition of the cell culture medium additive including the biocompatible polymer composition being set as 1. The evaluation results are shown in Table 5.
Ⓞ: 2 or higher (Very good)
○: 1 or higher to less than 2 (Good)
×: Less than 1 (Poor)

### (2) Evaluation of cell activity

### <ATP activity>

The medium composition was prepared by adding the cell culture medium additive containing the biocompatible polymer composition to 10% FBS-DMEM medium to achieve the concentration listed in Table 5, and then stirring. HepG2 cells, a human liver cancer cell line, were seeded to achieve 100,000 cells/mL in the medium composition containing the cell culture medium additive with the biocompatible polymer composition, and then dispensed into the wells of a 96-well U-bottom microplate at 100 µL per well. Then, the plate was cultured in a static state in a CO₂ incubator (37°C, 5% CO₂) for 5 days.

ATP activity was evaluated by performing ATP assay 1 day and 5 days after the culturing starts. Specifically, 100 µL of ATP reagent (KATAMARI ATP assay Kit: manufactured by Toyo B-Net Co., Ltd.) was added to the wells after cultivation, pipetted 5 times, and left at room temperature for 5 minutes. Then, 100 µL of the obtained reagent-cell dissolution mixture was taken into a separate plate and stirred for 1 minute. By using the plate, the luminescence was measured using MithrasLB940 (manufactured by Berthold). The evaluation results are shown in Table 5. ATP activity = (Luminescence after 5 days of culturing)/(Luminescence after 1 day of culturing) × 100
Ⓞ: 150% ≤ ATP activity, ATP activity is considerably high. (Very good)
O: 100% ≤ ATP activity < 150%, ATP activity is high. (Good)
Δ: 50% ≤ ATP activity < 100%, ATP activity is moderately high. (Acceptable)
×: ATP activity < 50%, ATP activity is low. (Poor)

**[Table 5]**

| Table 5 | | Concentration in culture medium [mass%] | (1) Evaluation of production amount of bioactive substance | | | | (2) Cell activity evaluation |
|---|---|---|---|---|---|---|---|
| | | | Antibody productivity | Albumin productivity | Drug metabolizing enzyme activity | Gene expression | |
| Example 4-1 | Cell culture medium additive 1 | 1 | ○ | Ⓞ | Ⓞ | Ⓞ | Ⓞ |
| | | 0.05 | ○ | ○ | ○ | ○ | Ⓞ |
| | | 0.01 | ○ | ○ | ○ | ○ | ○ |
| Example 4-2 | Cell culture medium additive 2 | 1 | Ⓞ | ○ | ○ | ○ | Ⓞ |
| | | 0.05 | ○ | ○ | ○ | ○ | Ⓞ |
| Example 4-3 | Cell culture medium additive 3 | 1 | Ⓞ | ○ | ○ | ○ | Ⓞ |
| | | 0.05 | ○ | ○ | ○ | ○ | Ⓞ |
| Example 4-4 | Cell culture medium additive 4 | 1 | Ⓞ | ○ | ○ | ○ | Ⓞ |
| | | 0.05 | ○ | ○ | ○ | ○ | Ⓞ |
| Example 4-5 | Cell culture medium additive 5 | 1 | Ⓞ | Ⓞ | Ⓞ | Ⓞ | Ⓞ |
| | | 0.05 | Ⓞ | Ⓞ | Ⓞ | Ⓞ | Ⓞ |
| Example 4-6 | Cell culture medium additive 6 | 1 | Ⓞ | ○ | ○ | ○ | Ⓞ |
| | | 0.05 | ○ | ○ | ○ | ○ | Ⓞ |
| Example 4-7 | Cell culture medium additive 7 | 1 | Ⓞ | ○ | ○ | ○ | Ⓞ |
| | | 0.05 | Ⓞ | ○ | ○ | ○ | Ⓞ |
| Example 4-8 | Cell culture medium additive 8 | 1 | Ⓞ | ○ | ○ | ○ | Ⓞ |
| | | 0.05 | Ⓞ | ○ | ○ | ○ | Ⓞ |
| Example 4-9 | Cell culture medium additive 9 | 1 | Ⓞ | Ⓞ | Ⓞ | Ⓞ | Ⓞ |
| | | 0.05 | Ⓞ | ○ | ○ | ○ | Ⓞ |
| Example 4-10 | Cell culture medium additive 10 | 1 | Ⓞ | Ⓞ | Ⓞ | Ⓞ | Ⓞ |
| | | 0.05 | Ⓞ | ○ | ○ | ○ | Ⓞ |
| Example 4-11 | Cell culture medium additive 11 | 1 | Ⓞ | Ⓞ | Ⓞ | Ⓞ | Ⓞ |
| | | 0.05 | ○ | Ⓞ | Ⓞ | Ⓞ | Ⓞ |
| Example 4-12 | Cell culture medium additive 12 | 1 | Ⓞ | Ⓞ | Ⓞ | Ⓞ | Ⓞ |
| | | 0.05 | ○ | Ⓞ | Ⓞ | Ⓞ | Ⓞ |
| Example 4-13 | Cell culture medium additive 13 | 1 | Ⓞ | Ⓞ | Ⓞ | Ⓞ | Ⓞ |
| | | 0.05 | Ⓞ | ○ | ○ | ○ | Ⓞ |
| Example 4-14 | Cell culture medium additive 14 | 1 | Ⓞ | Ⓞ | Ⓞ | Ⓞ | Ⓞ |
| | | 0.05 | Ⓞ | ○ | ○ | ○ | Ⓞ |
| Example 4-15 | Cell culture medium additive 15 | 1 | Ⓞ | Ⓞ | Ⓞ | Ⓞ | Ⓞ |
| | | 0.05 | Ⓞ | ○ | ○ | ○ | Ⓞ |
| Example 4-16 | Cell culture medium additive 16 | 1 | Ⓞ | ○ | ○ | ○ | Ⓞ |
| | | 0.05 | Ⓞ | ○ | ○ | ○ | Ⓞ |
| Example 4-17 | Cell culture medium additive 17 | 1 | Ⓞ | ○ | ○ | ○ | Ⓞ |
| | | 0.05 | ○ | ○ | ○ | ○ | Ⓞ |
| Example 4-18 | Cell culture medium additive 18 | 1 | Ⓞ | ○ | ○ | ○ | Ⓞ |
| | | 0.05 | Ⓞ | ○ | ○ | ○ | Ⓞ |
| Example 4-19 | Cell culture medium additive 19 | 1 | Ⓞ | ○ | ○ | ○ | Ⓞ |
| | | 0.05 | ○ | ○ | ○ | ○ | Ⓞ |
| Example 4-20 | Cell culture medium additive 20 | 1 | Ⓞ | ○ | ○ | ○ | Ⓞ |
| | | 0.05 | Ⓞ | ○ | ○ | ○ | Ⓞ |
| Example 4-21 | Cell culture medium additive 21 | 1 | Ⓞ | ○ | ○ | ○ | Ⓞ |
| | | 0.05 | ○ | ○ | ○ | ○ | Ⓞ |
| Example 4-22 | Cell culture medium additive 22 | 1 | Ⓞ | ○ | ○ | ○ | Ⓞ |
| | | 0.05 | ○ | ○ | ○ | ○ | Ⓞ |
| Comparative Example 4-1 | Cell culture medium additive 23 | 0.5 | ○ | × | × | × | ○ |
| Comparative Example 4-2 | Cell culture medium additive 24 | 0.5 | × | ○ | ○ | ○ | ○ |
| Comparative Example 4-3 | Cell culture medium additive 25 | 0.5 | × | ○ | ○ | ○ | ○ |
| Comparative Example 4-4 | Cell culture medium additive 26 | 0.5 | × | ○ | ○ | ○ | ○ |

From the results in Table 5, it can be seen that the cell culture medium additives of Examples 4-1 to 4-22 showed excellent antibody productivity, albumin productivity, and activities of drug-metabolizing enzymes, etc. On the other hand, the additives of Comparative Examples 4-1 to 4-4 showed inferior results in antibody productivity, albumin productivity, and ATP activity when compared to the cell culture medium additives of Examples 4-1 to 4-22.

The biocompatible polymer composition of the disclosure can be used as a protein stabilizer that enables stable storage without impairing the function of proteins when coexisting with proteins.

In addition, by using the biocompatible polymer composition as a blocking agent, it is possible to perform analyses utilizing the antigen-antibody reaction such as Western blotting, immunohistochemical staining, ELISA method, and immunochromatography method with high sensitivity.

Furthermore, by adding the biocompatible polymer composition to the cell culture medium, it is possible to increase the production amount of bioactive substances, and it is also useful in reducing manufacturing costs and labor. For example, in relation to the manufacture of biopharmaceuticals such as antibody drugs, it can greatly contribute to large-scale supply.

## Claims

1. A biocompatible polymer composition, comprising: a vinyl polymer (A) and water, the vinyl polymer having an amine oxide group and a mass-average molecular weight of 5,000 or more, wherein a surface tension of a 1% aqueous solution at 25°C is 30 dyne/cm to 50 dyne/cm.

2. The biocompatible polymer composition as claimed in claim 1, wherein an osmotic pressure in the 1% aqueous solution is 1 mOsm/kg to 20 mOsm/kg.

3. The biocompatible polymer composition as claimed in claim 1 or 2, further comprising a compound (B), excluding a surfactant and a monomer, having an amphoteric ion structure, and exhibiting a molecular weight of 500 or less.

4. The biocompatible polymer composition as claimed in claim 3, wherein a proportion of the compound (B) is 0.5 parts to 10 parts with respect to 100 parts of the vinyl polymer (A).

5. The biocompatible polymer composition as claimed in any one of claims 1 to 4, wherein the vinyl polymer (A) has at least one structure represented by General formulas 1 to 3 below 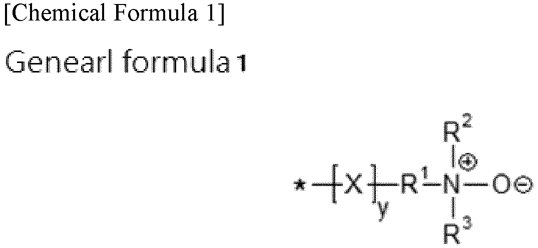 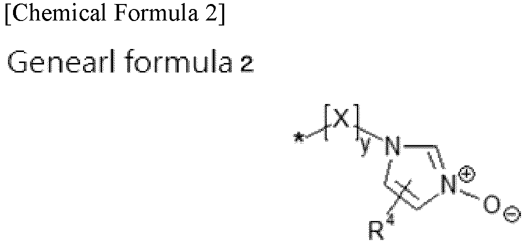 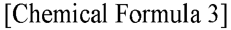 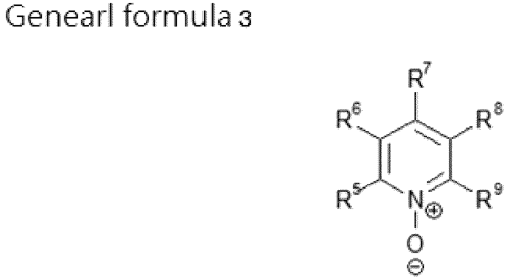 [In General formulas 1 to 3,
X represents a divalent linking group,
y represents 0 or 1,
R¹ represents an alkylene group having 1 to 6 carbon atoms,
R² and R³ each independently represent an alkyl group having 1 to 4 carbon atoms,
R⁴ represents a hydrogen atom or an alkyl group having 1 to 4 carbon atoms,
four of R⁵ to R⁹ each independently represent a hydrogen atom or an alkyl group having 1 to 6 carbon atoms, and one of R⁵ to R⁹ represents a bonding position with a main chain of the vinyl polymer (A), and
* represents the bonding position with the main chain of the vinyl polymer (A).

6. The biocompatible polymer composition as claimed in claim 3 or 4, wherein the compound (B) has at least one structure represented by General formulas 4 to 9 below: 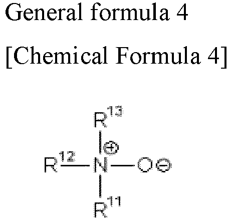 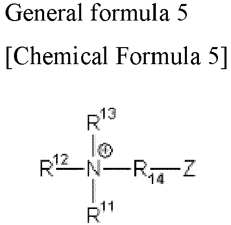 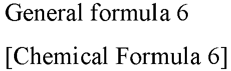 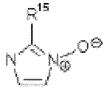 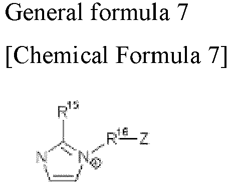 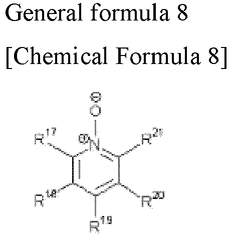 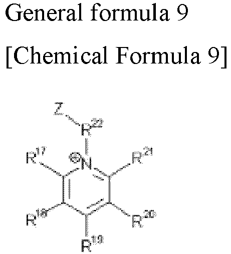 [wherein in General formulas 4 to 9,
R¹¹, R¹², and R¹³ each independently represent an alkyl group or a hydroxyalkyl group having 1 to 5 carbon atoms, any two of R¹¹, R¹², and R¹³ are linked to form a ring,
R¹⁴ represents an alkylene group having 1 to 4 carbon atoms,
Z represents -COO⁻ or SO3⁻,
R¹⁵ represents a hydrogen atom or a methyl group,
R¹⁶ represents an alkylene group having 1 to 4 carbon atoms,
R¹⁷ to R²¹ represent hydrogen atoms or alkyl groups having 1 to 6 carbon atoms, and
R²² represents an alkylene group having 1 to 4 carbon atoms.]

7. A protein stabilizer, comprising the biocompatible polymer composition as claimed in any one of claims 1 to 6.

8. A method for stabilizing a protein, allowing the protein stabilizer as claimed in claim 7 and a protein to coexist.

9. A blocking agent, comprising the biocompatible polymer composition as claimed in any one of claims 1 to 6.

10. A method for detecting a target substance in a sample through in-vitro diagnostics using the blocking agent as claimed in claim 9.

11. A cell culture medium additive, comprising the biocompatible polymer composition as claimed in any one of claims 1 to 6.

12. A cell culture medium, comprising the cell culture medium additive as claimed in claim 11.

13. The cell culture medium as claimed in claim 12, wherein a concentration of the biocompatible polymer composition in the cell culture medium is 0.01 mass% or more and 1 mass% or less.

14. A method for manufacturing a cell aggregate or a bioactive substance, the method comprising: culturing cells by using the cell culture medium as claimed in claim 12 or 13.
